# EUROPEAN PATENT APPLICATION

(11) **EP 4 428 120 A1**
(43) Date of publication of application: **11.09.2024**
(21) Application number: 22890041.1
(22) Date of filing: 04.11.2022
(51) Int. Cl.: C07D 211/46, C08F 220/36, C08K 5/3432, C08L 101/00, C09D 201/00, C09D 7/65

(54) **COMPOUND, POLYMER, PHOTOSTABILIZER COMPOSITION, RESIN COMPOSITION, COATING MATERIAL COMPOSITION, ARTICLE, SEALING MATERIAL, MOLDED ARTICLE, METHOD FOR PRODUCING WEATHER-RESISTANT RESIN COMPOSITION, AND METHOD FOR IMPROVING WEATHER RESISTANCE OF SYNTHETIC RESIN**

(30) Priority: 05.11.2021 JP 2021181270
(71) Applicant: ADEKA CORPORATION, Arakawa-ku Tokyo 116-8554 (JP)
(72) Inventor: ISHIZUKI Kazuya, Saitama-shi, Saitama 336-0022 (JP); MASAI Shogo, Saitama-shi, Saitama 336-0022 (JP)
(74) Representative: Gill Jennings & Every LLP
(86) International application number: PCT/JP2022/041271
(87) International publication number: WO 2023/080221

(57) **Abstract**

To provide a compound, a polymer, a light stabilizer composition, a resin composition, a coating material composition, an article, a sealing material, a molded article, a method for producing a weather-resistant resin composition, and a method for improving weather resistance of a synthetic resin, in which excellent weather resistance can be imparted to a resin composition. A compound represented by the following general formula (1'): 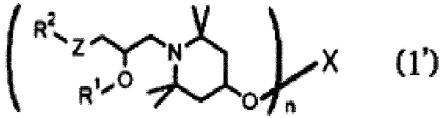 wherein n represents 1 or 2, when n is 1, X represents, for example, a substituted or unsubstituted monovalent hydrocarbon group having 1 to 30 carbon atoms, when n is 2, X represents, for example, a substituted or unsubstituted divalent hydrocarbon group having 1 to 30 carbon atoms and Y represents, for example, a single bond, Z represents, for example, a single bond, R¹ represents, for example, a hydrogen atom, R² represents, for example, a substituted or unsubstituted monovalent hydrocarbon group having 1 to 30 carbon atoms, and each of R³, R⁵ and R⁶ independently represents a substituted or unsubstituted monovalent hydrocarbon group having 1 to 30 carbon atoms.

## Description

### TECHNICAL FIELD

The present invention relates to a compound, a polymer, a light stabilizer composition, a resin composition, a coating material composition, an article, a sealing material, a molded article, a method for producing a weather-resistant resin composition, and a method for improving weather resistance of a synthetic resin, and specifically relates to a compound, a polymer, a light stabilizer composition, a resin composition, a coating material composition, an article, a sealing material, a molded article, a method for producing a weather-resistant resin composition, and a method for improving weather resistance of a synthetic resin, in which excellent weather resistance can be imparted to a resin composition.

### BACKGROUND ART

Resin compositions used for articles such as automobiles and building materials are demanded to have weather resistance. For example, Patent Document 1 described below proposes a hindered amine compound having a hydroxyl group, as a compound capable of imparting weather resistance to a resin composition.

### RELATED ART DOCUMENT

### PATENT DOCUMENT

[Patent Document 1]: JP2004-522791A

### SUMMARY OF THE INVENTION

### PROBLEMS TO BE SOLVED BY THE INVENTION

However, the compound proposed in Patent Document 1 described above has room for further improvement in terms of weather resistance.

An object of the present invention is to provide a compound, a polymer, a light stabilizer composition, a resin composition, a coating material composition, an article, a sealing material, a molded article, a method for producing a weather-resistant resin composition, and a method for improving weather resistance of a synthetic resin, by which excellent weather resistance can be imparted to a resin composition.

### MEANS FOR SOLVING THE PROBLEMS

The present inventors have made intensive studies in order to solve the above problems, and as a result, have found that the above problems can be solved by a compound having a specified structure, leading to the completion of the present invention.

Specifically, the present invention relates to a compound, a polymer, a light stabilizer composition, a resin composition, a coating material composition, an article, a sealing material, a molded article, a method for producing a weather-resistant resin composition, and a method for improving weather resistance of a synthetic resin, according to the following [1] to [17].
[1] A compound represented by the following general formula (1'). 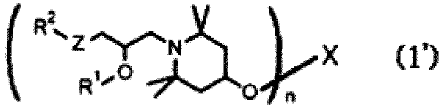 In the general formula (1'), n represents 1 or 2, when n is 1, X represents a substituted or unsubstituted monovalent hydrocarbon group having 1 to 30 carbon atoms, a group represented by -C(=O)-R³, or a group represented by the following general formula (2), when n is 2, X represents a substituted or unsubstituted divalent hydrocarbon group having 1 to 30 carbon atoms, a group represented by -C(=O)-Y-C(=O)-, or a -C(=O)- group and Y represents a single bond, or a substituted or unsubstituted divalent hydrocarbon group having 1 to 30 carbon atoms, Z represents a single bond or an oxygen atom, R¹ represents a hydrogen atom, a substituted or unsubstituted monovalent hydrocarbon group having 1 to 30 carbon atoms, or a group represented by -C(=O)-R⁵, R² represents a substituted or unsubstituted monovalent hydrocarbon group having 1 to 30 carbon atoms, or a group represented by -C(=O)-R⁶, and each of R³, R⁵ and R⁶ independently represents a substituted or unsubstituted monovalent hydrocarbon group having 1 to 30 carbon atoms. 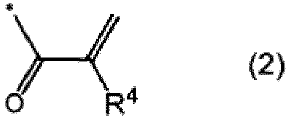 In the general formula (2), * represents a site bound with an oxygen atom and R⁴ represents a hydrogen atom, or a substituted or unsubstituted monovalent hydrocarbon group having 1 to 30 carbon atoms.
[2] The compound according to [1], wherein, in the general formula (1'), n represents 1 or 2, when n is 1, X represents a substituted or unsubstituted monovalent aliphatic hydrocarbon group having 1 to 30 carbon atoms, a group represented by -C(=O)-R³, or the group represented by the general formula (2), when n is 2, X represents a substituted or unsubstituted divalent aliphatic hydrocarbon group having 1 to 30 carbon atoms, a group represented by -C(=O)-Y-C(=O)-, or a -C(=O)- group and Y represents a single bond, or a substituted or unsubstituted divalent aliphatic hydrocarbon group having 1 to 30 carbon atoms, Z represents a single bond or an oxygen atom, R¹ represents a hydrogen atom, a substituted or unsubstituted monovalent aliphatic hydrocarbon group having 1 to 30 carbon atoms, or a group represented by -C(=O)-R⁵, R² represents a substituted or unsubstituted monovalent aliphatic hydrocarbon group having 1 to 30 carbon atoms, or a group represented by -C(=O)-R⁶, and each of R³, R⁵ and R⁶ independently represents a substituted or unsubstituted monovalent aliphatic hydrocarbon group having 1 to 30 carbon atoms, and in the general formula (2), * represents a site bound with an oxygen atom and R⁴ represents a hydrogen atom, or a substituted or unsubstituted monovalent aliphatic hydrocarbon group having 1 to 30 carbon atoms.
[3] The compound according to [1], wherein Z is an oxygen atom.
[4] The compound according to [2], wherein Z is an oxygen atom.
[5] The compound according to [1], represented by the following general formula (1). 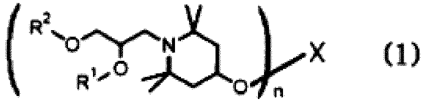 In the general formula (1), n represents 1 or 2, when n is 1, X represents a monovalent hydrocarbon group having 1 to 30 carbon atoms, a group represented by -C(=O)-R³, or a group represented by the following general formula (2), when n is 2, X represents a divalent hydrocarbon group having 1 to 30 carbon atoms, a group represented by -C(=O)-Y-C(=O)-, or a -C(=O)- group and Y represents a single bond or a divalent hydrocarbon group having 1 to 30 carbon atoms, R¹ represents a hydrogen atom, a monovalent hydrocarbon group having 1 to 30 carbon atoms or a group represented by -C(=O)-R⁵, and each of R², R³ and R⁵ independently represents a monovalent hydrocarbon group having 1 to 30 carbon atoms. 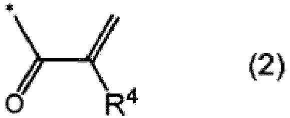 In the general formula (2), * represents a site bound with an oxygen atom and R⁴ represents a hydrogen atom or a monovalent hydrocarbon group having 1 to 30 carbon atoms.
[6] The compound according to [5], wherein, in the general formula (1), n represents 1 or 2, when n is 1, X represents a monovalent aliphatic hydrocarbon group having 1 to 30 carbon atoms, a group represented by -C(=O)-R³, or the group represented by the general formula (2), when n is 2, X represents a divalent aliphatic hydrocarbon group having 1 to 30 carbon atoms, a group represented by -C(=O)-Y-C(=O)-, or a -C(=O)- group and Y represents a single bond or a divalent aliphatic hydrocarbon group having 1 to 30 carbon atoms, R¹ represents a hydrogen atom, a monovalent aliphatic hydrocarbon group having 1 to 30 carbon atoms or a group represented by -C(=O)-R⁵, and each of R², R³ and R⁵ independently represents a monovalent aliphatic hydrocarbon group having 1 to 30 carbon atoms, and in the general formula (2), * represents a site bound with an oxygen atom and R⁴ represents a hydrogen atom or a monovalent aliphatic hydrocarbon group having 1 to 30 carbon atoms.
[7] A polymer obtained by polymerizing a monomer component comprising the compound according to any of [1] to [6] in which n is 1 and X is the group represented by the general formula (2).
[8] A light stabilizer composition comprising the compound according to any of [1] to [6].
[9] The light stabilizer composition according to [8], wherein the composition is a liquid.
[10] A light stabilizer composition comprising the polymer according to [7].
[11] A resin composition comprising a synthetic resin, and the compound according to any of [1] to [6] or the polymer according to [7].
[12] A coating material composition comprising the resin composition according to [11].
[13] An article comprising a coating film obtained by curing the coating material composition according to [12].
[14] A sealing material comprising the resin composition according to [11].
[15] A molded article obtained by molding the resin composition according to [11].
[16] A method for producing a weather-resistant resin composition, comprising a step of blending the compound according to any of [1] to [6] or the polymer according to [7], with a synthetic resin.
[17] A method for improving weather resistance of a synthetic resin, comprising a step of blending the compound according to any of [1] to [6] or the polymer according to [7], with the synthetic resin.

### EFFECTS OF THE INVENTION

According to the present invention, it is possible to provide a compound, a polymer, a light stabilizer composition, a resin composition, a coating material composition, an article, a sealing material, a molded article, a method for producing a weather-resistant resin composition, and a method for improving weather resistance of a synthetic resin, by which excellent weather resistance can be imparted to a resin composition.

### MODE FOR CARRYING OUT THE INVENTION

Hereinafter, embodiments of the present invention are described in detail. First, a compound of the present embodiment is described.

### <Compound>

The compound of the present embodiment is represented by the following general formula (1').

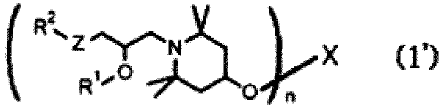

In the general formula (1'), n represents 1 or 2, when n is 1, X represents a substituted or unsubstituted monovalent hydrocarbon group having 1 to 30 carbon atoms, a group represented by -C(=O)-R³, or a group represented by the following general formula (2), when n is 2, X represents a substituted or unsubstituted divalent hydrocarbon group having 1 to 30 carbon atoms, a group represented by -C(=O)-Y-C(=O)-, or a -C(=O)- group and Y represents a single bond, or a substituted or unsubstituted divalent hydrocarbon group having 1 to 30 carbon atoms, Z represents a single bond or an oxygen atom, R¹ represents a hydrogen atom, a substituted or unsubstituted monovalent hydrocarbon group having 1 to 30 carbon atoms, or a group represented by -C(=O)-R⁵, R² represents a substituted or unsubstituted monovalent hydrocarbon group having 1 to 30 carbon atoms, or a group represented by -C(=O)-R⁶, and each of R³, R⁵ and R⁶ independently represents a substituted or unsubstituted monovalent hydrocarbon group having 1 to 30 carbon atoms.

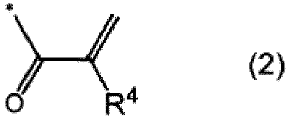

In the general formula (2), * represents a site bound with an oxygen atom and R⁴ represents a hydrogen atom, or a substituted or unsubstituted monovalent hydrocarbon group having 1 to 30 carbon atoms.

The compound of the present embodiment can impart weather resistance to a resin composition.

Examples of the substituted or unsubstituted monovalent hydrocarbon group having 1 to 30 carbon atoms, represented by X, include a substituted or unsubstituted monovalent aliphatic saturated hydrocarbon group, a substituted or unsubstituted monovalent aliphatic unsaturated hydrocarbon group, and a substituted or unsubstituted monovalent aromatic hydrocarbon group. Examples of the monovalent aliphatic saturated hydrocarbon group here include linear alkyl groups such as a methyl group, an ethyl group, a n-propyl group, a n-butyl group, a n-pentyl group, a n-hexyl group, a n-heptyl group, a n-octyl group, a n-nonyl group, a n-decyl group, a n-dodecyl group, a n-tetradecyl group, a n-hexadecyl group, a n-octadecyl group, a n-eicosyl group, a n-docosyl group and n-triacontyl, branched alkyl groups such as an isopropyl group, an isobutyl group, a sec-butyl group, a tert-butyl group, an isopentyl group, a neopentyl group, a tert-amyl group, a 2-heptyl group, a tert-heptyl group, a 2-ethylhexyl group, a tert-octyl group, an isononyl group, an isodecyl group and a 3,5,5-trimethylhexyl group, and cyclic alkyl groups such as a cyclopentyl group, a cyclohexyl group, a cyclooctyl group, and an adamantyl group. Examples of the monovalent aliphatic unsaturated hydrocarbon group include a 9-octadecenyl group and a 9,12-octadecadienyl group. Examples of the monovalent aromatic hydrocarbon group include a phenyl group, a naphthyl group, a biphenylyl group, and an anthracyl group.

In a case where the monovalent hydrocarbon having 1 to 30 carbon atoms, represented by X, has a substituent, examples of the substituent include halogen atoms such as a fluorine atom, a chlorine atom and a bromine atom, alkoxy groups such as a methoxy group, an ethoxy group and a tert-butyloxy group, trialkylsilyl groups such as a trimethylsilyl group and a triethylsilyl group, dialkylalkoxysilyl groups such as a dimethylmethoxysilyl group, a dimethylethoxysilyl group, a diethylmethoxysilyl group and a diethylethoxysilyl group, alkyldialkoxysilyl groups such as a methyldimethoxysilyl group, a methyldiethoxysilyl group, an ethyldimethoxysilyl group and an ethyldiethoxysilyl group, trialkoxysilyl groups such as a trimethoxysilyl group and a triethoxysilyl group, trialkylsiloxy groups such as a trimethylsiloxy group and a triethylsiloxy group, dialkylalkoxysiloxy groups such as a dimethylmethoxysiloxy group, a dimethylethoxysiloxy group, a diethylmethoxysiloxy group and a diethylethoxysiloxy group, alkyldialkoxysiloxy groups such as a methyldimethoxysiloxy group, a methyldiethoxysiloxy group, an ethyldimethoxysiloxy group and an ethyldiethoxysiloxy group, and trialkoxysiloxy groups such as a trimethoxysiloxy group and a triethoxysiloxy group. In particular, alkoxy groups, trialkylsilyl groups, trialkoxysilyl groups, trialkylsiloxy groups, and trialkoxysiloxy groups are preferred, trialkylsilyl groups, trialkoxysilyl groups, trialkylsiloxy groups, and trialkoxysiloxy groups are more preferred, trialkylsilyl groups and trialkoxysilyl groups are further preferred, and trialkoxysilyl groups are particularly preferred.

Examples of the substituted or unsubstituted divalent hydrocarbon group having 1 to 30 carbon atoms, represented by X, include substituted or unsubstituted divalent aliphatic saturated hydrocarbon groups, substituted or unsubstituted divalent aliphatic unsaturated hydrocarbon groups, and substituted or unsubstituted divalent aromatic hydrocarbon groups. Examples of the divalent saturated hydrocarbon group include linear alkanediyl groups such as a methylene group, an ethylene group, a propylene group, a butylene group, a hexylene group, an octylene group, a decylene group, a dodecylene group, a hexadecylene group, octadecylene group, an eicosylene group, a docosylene group, a triacontylene group, an ethan-1,1-diyl group, a propan-1,1-diyl group, a butan-1,1-diyl group and a hexan-1,1-diyl group, branched alkanediyl groups such as a propan-2,2-diyl group, and cyclic alkanediyl groups such as a cyclohexan-1,4-diyl group and a cyclohexan-1,1-diyl group. Examples of the divalent unsaturated hydrocarbon group include a 9-octadecen-1,18-diyl group and a 9,12-octadecadien-1,18-diyl group. Examples of the divalent aromatic hydrocarbon group include a phenylene group, a naphthylene group, and a biphenylene group.

In a case where the divalent hydrocarbon having 1 to 30 carbon atoms, represented by X, has a substituent, examples of the substituent include the same as substituents exemplified for a case where the monovalent hydrocarbon having 1 to 30 carbon atoms, represented by X, has a substituent.

Examples of the substituted or unsubstituted divalent hydrocarbon group having 1 to 30 carbon atoms, represented by Y, include the same as those exemplified for the substituted or unsubstituted divalent hydrocarbon group having 1 to 30 carbon atoms, represented by X.

Examples of the substituted or unsubstituted monovalent hydrocarbon group having 1 to 30 carbon atoms, represented by R¹ to R⁶, include the same as those for the substituted or unsubstituted monovalent hydrocarbon group having 1 to 30 carbon atoms, represented by X.

In the compound of the present embodiment, preferably, when n is 1, X represents a substituted or unsubstituted monovalent aliphatic hydrocarbon group having 1 to 30 carbon atoms, a group represented by -C(=O)-R³, or the group represented by the general formula (2), when n is 2, X represents a substituted or unsubstituted divalent aliphatic hydrocarbon group having 1 to 30 carbon atoms, a group represented by -C(=O)-Y-C(=O)-, or a -C(=O)-group and Y represents a single bond, or a substituted or unsubstituted divalent aliphatic hydrocarbon group having 1 to 30 carbon atoms, R¹ represents a hydrogen atom, a substituted or unsubstituted monovalent aliphatic hydrocarbon group having 1 to 30 carbon atoms, or a group represented by -C(=O)-R⁵, R² represents a substituted or unsubstituted monovalent aliphatic hydrocarbon group having 1 to 30 carbon atoms, or a group represented by -C(=O)-R⁶, each of R³, R⁵ and R⁶ independently represents a substituted or unsubstituted monovalent aliphatic hydrocarbon group having 1 to 30 carbon atoms, and R⁴ represents a hydrogen atom, or a substituted or unsubstituted monovalent aliphatic hydrocarbon group having 1 to 30 carbon atoms, from the viewpoint of allowing more excellent weather resistance to be imparted to a resin composition.

Z is preferably an oxygen atom from the same viewpoint.

Z may also be a single bond. In other words, the compound of the present embodiment may also be one represented by the following general formula (1").

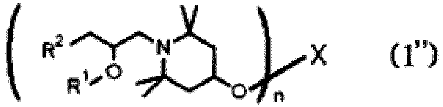

In the general formula (1 "), n represents 1 or 2, when n is 1, X represents a substituted or unsubstituted monovalent hydrocarbon group having 1 to 30 carbon atoms, a group represented by -C(=O)-R³, or the group represented by the general formula (2), when n is 2, X represents a substituted or unsubstituted divalent hydrocarbon group having 1 to 30 carbon atoms, a group represented by -C(=O)-Y-C(=O)-, or a -C(=O)- group and Y represents a single bond, or a substituted or unsubstituted divalent hydrocarbon group having 1 to 30 carbon atoms, R¹ represents a hydrogen atom, a substituted or unsubstituted monovalent hydrocarbon group having 1 to 30 carbon atoms, or a group represented by -C(=O)-R⁵, R² represents a substituted or unsubstituted monovalent hydrocarbon group having 1 to 30 carbon atoms, or a group represented by -C(=O)-R⁶, and each of R³, R⁵ and R⁶ independently represents a substituted or unsubstituted monovalent hydrocarbon group having 1 to 30 carbon atoms.

In the compound of the present embodiment, when n is 1, X preferably represents a group represented by -C(=O)-R³, or the group represented by the general formula (2), more preferably a group represented by -C(=O)-R³, from the viewpoint of allowing more excellent weather resistance to be imparted to a resin composition. When n is 2, X preferably represents a group represented by -C(=O)-Y-C(=O)- or a -C(=O)- group, more preferably a group represented by -C(=O)-Y-C(=O)-, from the same viewpoint.

Y preferably represents a substituted or unsubstituted divalent hydrocarbon group having 1 to 30 carbon atoms, more preferably represents a substituted or unsubstituted divalent aliphatic hydrocarbon group having 1 to 30 carbon atoms, still more preferably represents a substituted or unsubstituted divalent aliphatic saturated hydrocarbon group having 1 to 30 carbon atoms, further preferably represents an unsubstituted divalent aliphatic saturated hydrocarbon group having 1 to 30 carbon atoms, still further preferably represents a linear alkanediyl group having 1 to 30 carbon atoms, particularly preferably represents an alkylene group having 1 to 30 carbon atoms, from the viewpoint of allowing further excellent weather resistance to be imparted to a resin composition. The number of carbon atoms in Y is preferably 2 to 16, more preferably 4 to 14, further preferably 6 to 12 from the viewpoint of allowing further excellent weather resistance to be imparted to a resin composition.

When n is 1, X may represent the group represented by the general formula (2). In this case, the compound of the present embodiment contains a site having polymerization activity and thus can serve as a monomer component of a homopolymer or a copolymer.

In the compound of the present embodiment, R¹ preferably represents a hydrogen atom or a group represented by -C(=O)-R⁵, more preferably represents a hydrogen atom, from the viewpoint of allowing more excellent weather resistance to be imparted to a resin composition. R¹ may represent a group represented by -C(=O)-R⁵.

R² preferably represents a substituted or unsubstituted monovalent hydrocarbon group having 1 to 30 carbon atoms, more preferably represents a substituted or unsubstituted monovalent aliphatic hydrocarbon group having 1 to 30 carbon atoms, still more preferably represents a substituted or unsubstituted monovalent aliphatic saturated hydrocarbon group having 1 to 30 carbon atoms, further preferably represents an unsubstituted monovalent aliphatic saturated hydrocarbon group having 1 to 30 carbon atoms, still further preferably a linear alkyl group having 1 to 30 carbon atoms from the same viewpoint. The number of carbon atoms in R² is preferably 1 to 20, more preferably 1 to 10, further preferably 1 to 5 from the same viewpoint. Herein, R² may represent a group represented by -C(=O)-R⁶.

R³ preferably represents a substituted or unsubstituted monovalent aliphatic hydrocarbon group having 1 to 30 carbon atoms, more preferably represents a substituted or unsubstituted monovalent aliphatic saturated hydrocarbon group having 1 to 30 carbon atoms, further preferably represents an unsubstituted monovalent aliphatic saturated hydrocarbon group having 1 to 30 carbon atoms, still further preferably a linear alkyl group having 1 to 30 carbon atoms from the same viewpoint. The number of carbon atoms in R³ is preferably 5 to 23, more preferably 7 to 21, further preferably 9 to 19, still further preferably 11 to 17, from the same viewpoint. Furthermore, R³ may represent a branched alkyl group having 1 to 30 carbon atoms.

R⁴ preferably represents a substituted or unsubstituted monovalent hydrocarbon group having 1 to 30 carbon atoms, more preferably represents a substituted or unsubstituted monovalent aliphatic hydrocarbon group having 1 to 30 carbon atoms, still more preferably represents a substituted or unsubstituted monovalent aliphatic saturated hydrocarbon group having 1 to 30 carbon atoms, further preferably represents an unsubstituted monovalent aliphatic saturated hydrocarbon group having 1 to 30 carbon atoms, still further preferably a linear alkyl group having 1 to 30 carbon atoms from the same viewpoint. The number of carbon atoms in R⁴ is preferably 1 to 4, more preferably 1 to 2, further preferably 1 from the same viewpoint. Herein, R⁴ may represent a hydrogen atom.

Each of R⁵ and R⁶ preferably represents a substituted or unsubstituted monovalent aliphatic hydrocarbon group having 1 to 30 carbon atoms, more preferably represents a substituted or unsubstituted monovalent aliphatic saturated hydrocarbon group having 1 to 30 carbon atoms, further preferably represents an unsubstituted monovalent aliphatic saturated hydrocarbon group having 1 to 30 carbon atoms, still further preferably represents a linear alkyl group having 1 to 30 carbon atoms or a branched alkyl group having 1 to 30 carbon atoms from the same viewpoint. The number of carbon atoms in R⁵ is preferably 5 to 23, more preferably 7 to 21, further preferably 9 to 19, still further preferably 11 to 17 from the same viewpoint. R⁵ may represent a vinyl group, or a 1-alkylethenyl group such as a 1-methylethenyl group or a 1-ethylethenyl group.

The compound of the present embodiment may also be one represented by the following general formula (1).

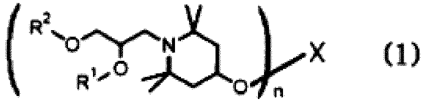

In the general formula (1), n represents 1 or 2, when n is 1, X represents a monovalent hydrocarbon group having 1 to 30 carbon atoms, a group represented by -C(=O)-R³, or the group represented by the general formula (2), when n is 2, X represents a divalent hydrocarbon group having 1 to 30 carbon atoms, a group represented by -C(=O)-Y-C(=O)-, or a -C(=O)- group and Y represents a single bond or a divalent hydrocarbon group having 1 to 30 carbon atoms, R¹ represents a hydrogen atom, a monovalent hydrocarbon group having 1 to 30 carbon atoms or a group represented by -C(=O)-R⁵, and each of R², R³ and R⁵ independently represents a monovalent hydrocarbon group having 1 to 30 carbon atoms.

Specific examples of the compound of the present embodiment include the following compounds. It is noted that the compound of the present embodiment is not limited to the following compounds.

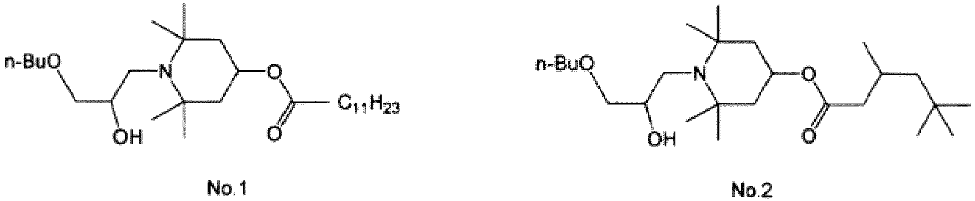

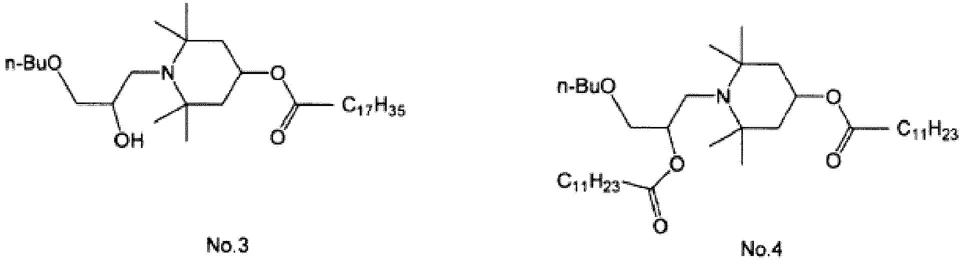

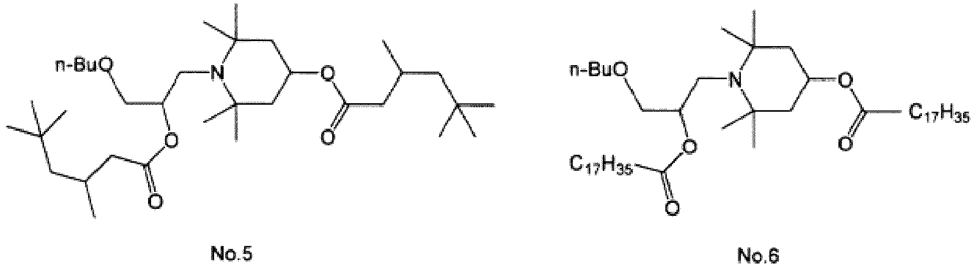

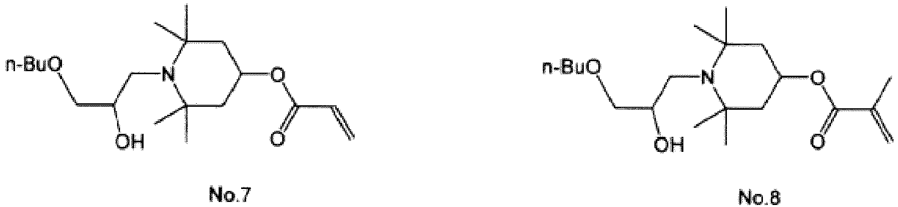

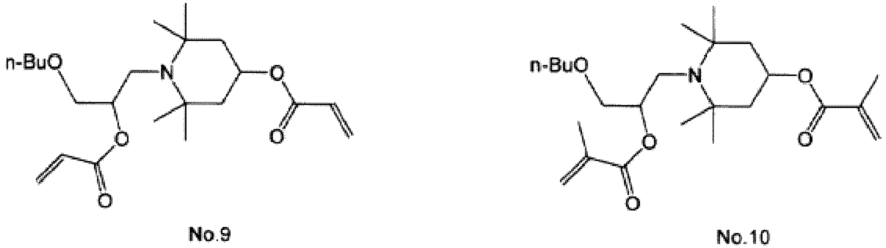

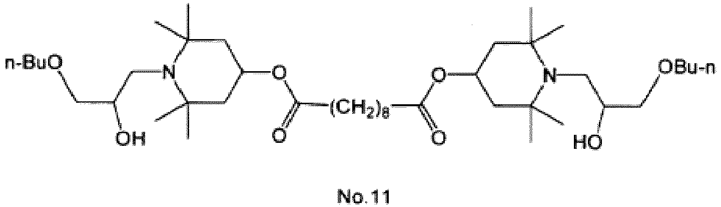

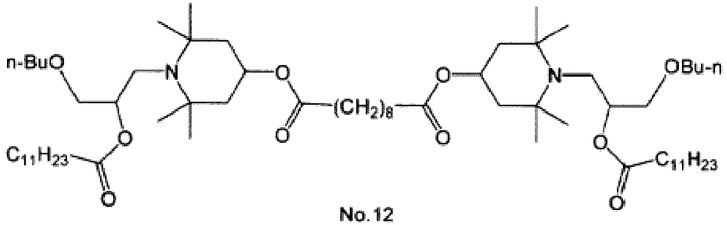

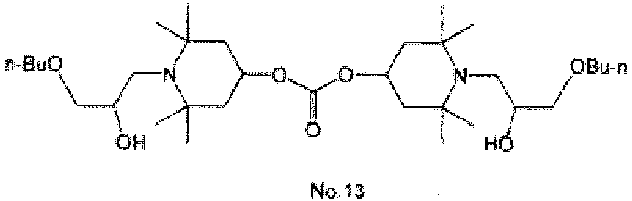

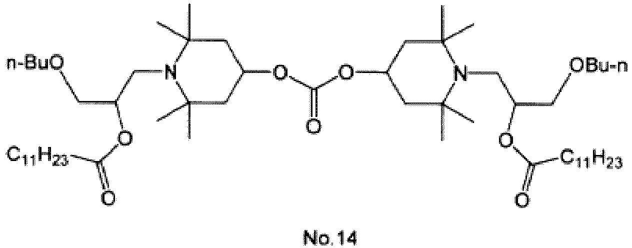

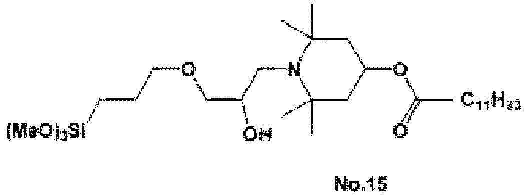

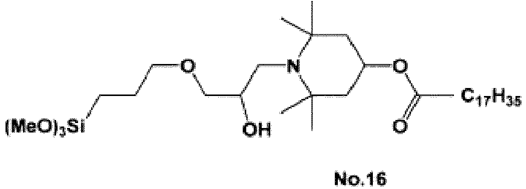

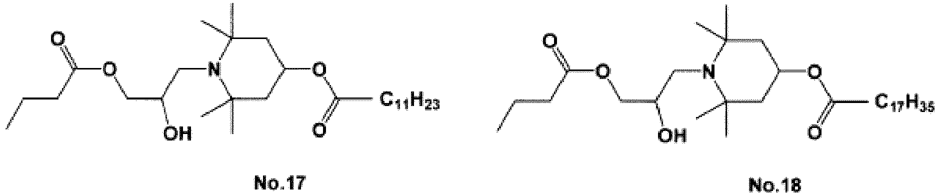

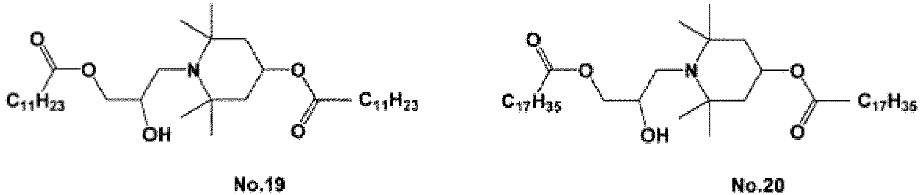

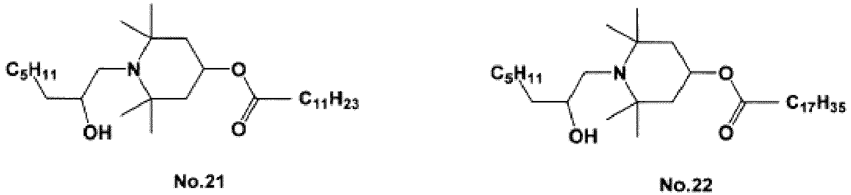

The compound of the present embodiment can be produced by a conventionally known method with, for example, an ester of 2,2,6,6-tetramethylpiperidin-4-ol, or monocarboxylic acid or dicarboxylic acid, and 2,2,6,6-tetramethylpiperidin-4-ol, as a starting material. For example, a compound in which n is 1, X represents a group represented by - C(=O)-R³, R¹ represents a hydrogen atom, and each of R² and R³ represents an alkyl group in the general formula (1) (the compound No. 1, No.2, No.3, or the like) can be produced by a method in which a monoester of 2,2,6,6-tetramethylpiperidin-4-ol and saturated fatty acid, as a starting material, is reacted with an alkyl glycidyl ether in the presence of a catalyst such as zinc chloride.

The compound of the present embodiment can be suitably used as a component of an additive composition for coating materials or an additive composition for resins, such as a light stabilizer composition, a flame retardant composition, or an antioxidant composition. The compound of the present embodiment can impart excellent weather resistance to a resin composition, and thus can be suitably used as a component of a light stabilizer composition.

The compound of the present embodiment may be any of a liquid and a solid, and is preferably a liquid. In this case, the compound is excellent in dispersibility and is suitable particularly as a component of an additive composition for coating materials. In a case where the compound of the present embodiment is a liquid, the compound of the present embodiment can be added with a liquid addition apparatus, and thus the compound of the present embodiment is excellent in handleability when used as a component of an additive composition for coating materials. In the present embodiment, the compound or the like "being a liquid" indicates that the compound or the like is a liquid under an environment at 1 atm and a temperature of 25°C.

Next, a polymer of the present embodiment is described.

### <Polymer>

The polymer of the present embodiment is obtained by polymerizing a monomer component including the compound which is represented by the general formula (1') and in which n is 1 and X is the group represented by the general formula (2).

The polymer of the present embodiment has excellent weather resistance.

The polymer may be obtained by polymerizing a monomer component including only the above compound, or may be obtained by polymerizing a monomer component further including other polymerizable compound in addition to the above compound. Examples of such other polymerizable compound include acrylates such as methyl acrylate, ethyl acrylate, 2-hydroxyethyl acrylate, propyl acrylate, isopropyl acrylate, 2-hydroxypropyl acrylate, butyl acrylate, isobutyl acrylate and 4-hydroxybutyl acrylate, methacrylates such as methyl methacrylate, ethyl methacrylate, 2-hydroxyethyl methacrylate, propyl methacrylate, isopropyl methacrylate, 2-hydroxypropyl methacrylate, butyl methacrylate, isobutyl methacrylate and 4-hydroxybutyl methacrylate, ethylene, propylene, 1-butene, butadiene, chloroethylene, styrene, acrylonitrile, and vinyl acetate. In particular, acrylates and methacrylates are preferred.

The method for polymerizing the monomer component is not particularly limited, and examples thereof include known methods such as solution polymerization, emulsion polymerization, and UV polymerization. The polymerization initiator is also not particularly limited, and examples thereof include an azo-based polymerization initiator such as 2,2'-azobisisobutyronitrile and a peroxide-based polymerization initiator such as benzoyl peroxide. The weight average molecular weight of the polymer is not particularly limited, and may be, for example, 2,000 to 200,000. In the present embodiment, the "weight average molecular weight" means the weight average molecular weight in terms of standard polystyrene, measured by a gel permeation chromatography (GPC) method.

Next, a light stabilizer composition of the present embodiment is described.

### <Light stabilizer composition>

The light stabilizer composition of the present embodiment includes the above compound or the above polymer. The light stabilizer composition of the present embodiment may include both the above compound and the above polymer.

The light stabilizer composition of the present embodiment can impart excellent weather resistance to a resin composition.

The light stabilizer composition of the present embodiment may include a hindered amine compound other than the above compound or the above polymer (hereinafter, referred to as "other hindered amine compound"), an ultraviolet absorber, a benzoate compound, a phenol-based antioxidant, a phosphorus-based antioxidant, and/or the like, other than the above compound or the above polymer.

Examples of such other hindered amine compound include 2,2,6,6-tetramethyl-4-piperidyl stearate, 1,2,2,6,6-pentamethyl-4-piperidyl stearate, 2,2,6,6-tetramethyl-4-piperidyl benzoate, bis(2,2,6,6-tetramethyl-4-piperidyl)sebacate, tetrakis(2,2,6,6-tetramethyl-4-piperidyl)-1,2,3,4-butane tetracarboxylate, tetrakis(1,2,2,6,6-pentamethyl-4-piperidyl)-1,2,3,4-butane tetracarboxylate, bis(2,2,6,6-tetramethyl-4-piperidyl)-di(tridecyl)-1,2,3,4-butane tetracarboxylate, bis(1,2,2,6,6-pentamethyl-4-piperidyl)-di(tridecyl)-1,2,3,4-butane tetracarboxylate, bis(1,2,2,4,4-pentamethyl-4-piperidyl)-2-butyl-2-(3,5-di-tert-butyl-4-hydroxybenzyl)malonate, a 1-(2-hydroxyethyl)-2,2,6,6-tetramethyl-4-piperidinol/diethyl succinate polycondensate, a 1,6-bis(2,2,6,6-tetramethyl-4-piperidylamino)hexane/2,4-dichloro-6-morpholino-s-triazine polycondensate, a 1,6-bis(2,2,6,6-tetramethyl-4-piperidylamino)hexane/2,4-dichloro-6-tert-octylamino-s-triazine polycondensate, 1,5,8,12-tetrakis[2,4-bis(N-butyl-N-(2,2,6,6-tetramethyl-4-piperidyl)amino)-s-triazin-6-yl]-1,5,8,12-tetraazadodecane, 1,5,8,12-tetrakis[2,4-bis(N-butyl-N-(1,2,2,6,6-pentamethyl-4-piperidyl)amino)-s-triazin-6-yl]-1,5,8-12-tetraazadodecane, 1,6,11-tris[2,4-bis(N-butyl-N-(2,2,6,6-tetramethyl-4-piperidyl)amino)-s-triazin-6-ylamino]undecane, 1,6,11-tris[2,4-bis(N-butyl-N-(1,2,2,6,6-pentamethyl-4-piperidyl)amino)-s-triazin-6-ylamino]undecane, 3,9-bis[1,1 -dimethyl-2-{tris(2,2,6,6-tetramethyl-4-piperidyloxycarbonyl)butylcarbonyloxy}ethyl]-2,4,8,10-tetraoxaspiro[5.5]undecane, 3,9-bis[1,1-dimethyl-2-{tris(1,2,2,6,6-pentamethyl-4-piperidyloxycarbonyl)butylcarbonyloxy}ethyl]-2,4,8,10-tetraoxaspiro[5.5]undecane, bis(1-undecyloxy-2,2,6,6-tetramethylpiperidin-4-yl)carbonate, 2,2,6,6-tetramethyl-4-piperidyl hexadecanoate, and 2,2,6,6-tetramethyl-4-piperidyl octadecanoate.

Examples of the ultraviolet absorber include 2-hydroxybenzophenones such as 2,4-dihydroxybenzophenone and 5,5'-methylenebis(2-hydroxy-4-methoxybenzophenone); 2-(2-hydroxyphenyl)benzotriazoles such as 2-(2-hydroxy-5-methylphenyl)benzotriazole, 2-(2-hydroxy-5-tert-octylphenyl)benzotriazole, 2-(2-hydroxy-3,5-di-tert-butylphenyl)-5-chlorobenzotriazole, 2-(2-hydroxy-3-tert-butyl-5-methylphenyl)-5-chlorobenzotriazole, 2-(2-hydroxy-3,5-dicumylphenyl)benzotriazole, 2,2'-methylenebis(4-tert-octyl-6-benzotriazolylphenol), polyethylene glycol ester of 2-(2-hydroxy-3-tert-butyl-5-carboxyphenyl)benzotriazole, 2-[2-hydroxy-3-(2-acryloyloxyethyl)-5-methylphenyl]benzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-octylphenyl]benzotriazole, 2-[2-hydroxy-3-(2-methacryloyloxyethyl)-5-tert-butylphenyl]-5-chlorobenzotriazole, 2-[2-hydroxy-5-(2-methacryloyloxyethyl)phenyl]benzotriazole, 2-[2-hydroxy-3-tert-butyl-5-(2-methacryloyloxyethyl)phenyl]benzotriazole, 2-[2-hydroxy-3-tert-amyl-5-(2-methacryloyloxyethyl)phenyl]benzotriazole, 2-[2-hydroxy-3-tert-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(3-methacryloyloxy-2-hydroxypropyl)phenyl]benzotriazole, and 2-[2-hydroxy-4-(3-methacryloyloxypropyl)phenyl]benzotriazole; substituted oxanilides such as 2-ethyl-2'-ethoxy oxanilide and 2-ethoxy-4'-dodecyl oxanilide; cyano acrylates such as ethyl-α-cyano-β,β-diphenyl acrylate and methyl-2-cyano-3-methyl-3-(p-methoxyphenyl)acrylate; triazines such as 2-(2-hydroxy-4-hexyloxyphenyl)-4,6-diphenyl-1,3,5-triazine, 2-(2-hydroxy-4-octyloxyphenyl)-4,6-bis(2,4-dimethylphenyl)-1,3,5-triazine, trioctyl-2,2',2"-((1,3,5-triazin-2,4,6-triyl)tris(3-hydroxybenzen-4-,1-diyl)tripropionate), 2-[2-hydroxy-4-{2-(2-ethylhexanoyloxy)ethoxy}phenyl]-4,6-diphenyl-1,3,5-triazine, 2,4,6-tris(2-hydroxy-4-hexyloxy-3-methylphenyl)-1,3,5-triazine, and 1,12-bis[2-[4-(4,6-diphenyl-1,3,5-triazin-2-yl)-3-hydroxyphenoxy]ethyl]dodecanedioate; and various metal salts or metal chelates, in particular, salts or chelates of nickel or chromium. In particular, triazines are particularly preferred. Among such triazines, 2-(2-hydroxy-4-hexyloxyphenyl)-4,6-diphenyl-1,3,5-triazine, 2-(2-hydroxy-4-octyloxyphenyl)-4,6-bis(2,4-dimethylphenyl)-1,3,5-triazine, 2-[2-hydroxy-4-{2-(2-ethylhexanoyloxy)ethoxy}phenyl]-4,6-diphenyl-1,3,5-triazine, and 2,4,6-tris(2-hydroxy-4-hexyloxy-3-methylphenyl)-1,3,5-triazine are preferred, 2-(2-hydroxy-4-hexyloxyphenyl)-4,6-diphenyl-1,3,5-triazine and 2-(2-hydroxy-4-octyloxyphenyl)-4,6-bis(2,4-dimethylphenyl)-1,3,5-triazine are more preferred, and 2-(2-hydroxy-4-octyloxyphenyl)-4,6-bis(2,4-dimethylphenyl)-1,3,5-triazine is further preferred.

Examples of the benzoate compound include phenyl salicylate, resorcinol monobenzoate, 2,4-di-tert-butylphenyl-3,5-di-tert-butyl-4-hydroxybenzoate, octyl(3,5-di-tert-butyl-4-hydroxy)benzoate, dodecyl(3,5-di-tert-butyl-4-hydroxy)benzoate, tetradecyl(3,5-di-tert-butyl-4-hydroxy)benzoate, hexadecyl(3,5-di-tert-butyl-4-hydroxy)benzoate, octadecyl(3,5-di-tert-butyl-4-hydroxy)benzoate, and behenyl(3,5-di-tert-butyl-4-hydroxy)benzoate.

Examples of the phenol-based antioxidant include 2,6-di-tert-butyl-4-ethylphenol, 2-tert-butyl-4,6-dimethylphenol, styrenated phenol, 2,2'-methylenebis(4-ethyl-6-tertbutylphenol), 2,2'-thiobis-(6-tert-butyl-4-methylphenol), 2,2'-thiodiethylenebis[3-(3,5-di-tert-butyl-4-hydroxyphenyl)propionate], 2-methyl-4,6-bis(octylsulfanylmethyl)phenol, 2,2'-isobutylidenebis(4,6-dimethylphenol), isooctyl-3-(3,5-di-tert-butyl-4-hydroxyphenyl)propionate, N,N'-hexan-1,6-diylbis[3-(3,5-di-tert-butyl-4-hydroxyphenyl)propionamide], 2,2'-oxamide-bis[ethyl-3-(3,5-di-tert-butyl-4-hydroxyphenyl)propionate], 2-ethylhexyl-3-(3',5'-di-tert-butyl-4'-hydroxyphenyl)propionate, 2,2'-ethylenebis(4,6-di-tert-butylphenol), ester of 3,5-di-tert-butyl-4-hydroxybenzenepropanoic acid and C13-15 alkyl, 2,5-di-tert-amylhydroquinone, a polymerized product of hindered phenol (trade name "AO.OH.98" manufactured by ADEKA POLYMER ADDITIVES EUROPE SAS), 2,2'-methylenebis[6-(1-methylcyclohexyl)-p-cresol], 2-tert-butyl-6-(3-tert-butyl-2-hydroxy5-methylbenzyl)-4-methylphenyl acrylate, 2-[1-(2-hydroxy-3,5-di-tert-pentylphenyl)ethyl]-4,6-di-tert-pentylphenyl acrylate, 6-[3-(3-tert-butyl-4-hydroxy-5-methyl)propoxy]-2,4,8,10-tetra-tert-butylbenz[d,f][1,3,2]-dioxaphosphobin, hexamethylenebis[3-(3,5-di-tert-butyl-4-hydroxyphenyl)propionate], a bis[monoethyl(3,5-di-tert-butyl-4-hydroxybenzyl)phosphonate]calcium salt, a reaction product of 5,7-bis(1,1-dimethylethyl)-3-hydroxy-2(3H)-benzofuranone and o-xylene, 2,6-di-tert-butyl-4-(4,6-bis(octylthio)-1,3,5-triazin-2-ylamino)phenol, DL-a-tocopherol (vitamin E), 2,6-bis(α-methylbenzyl)-4-methylphenol, bis[3,3-bis-(4'-hydroxy-3'-tert-butylphenyl)butanoic acid]glycol ester, 2,6-di-tert-butyl-p-cresol, 2,6-diphenyl-4-octadecyloxyphenol, stearyl(3,5-di-tert-butyl-4-hydroxyphenyl)propionate, distearyl(3,5-di-tert-butyl-4-hydroxybenzyl)phosphonate, tridecyl-3,5-tert-butyl-4-hydroxybenzyl thioacetate, thiodiethylenebis[(3,5-di-tert-butyl-4-hydroxyphenyl)propionate], 4,4'-thiobis(6-tert-butyl-m-cresol), 2-octylthio-4,6-di(3,5-di-tert-butyl-4-hydroxyphenoxy)-s-triazine, 2,2'-methylenebis(4-methyl-6-tert-butylphenol), bis[3,3-bis(4-hydroxy-3-tert-butylphenyl)butyric acid]glycol ester, 4,4'-butylidenebis(2,6-di-tert-butylphenol), 4,4'-butylidenebis(6-tert-butyl-3-methylphenol), 2,2'-ethylidenebis(4,6-di-tert-butylphenol), 1,1,3-tris(2-methyl-4-hydroxy-5-tert-butylphenyl)butane, bis[2-tert-butyl-4-methyl-6-(2-hydroxy-3-tert-butyl-5-methylbenzyl)phenyl]terephthalate, 1,3,5-tris(2,6-dimethyl-3-hydroxy-4-tert-butylbenzyl)isocyanurate, 1,3,5-tris(3,5-di-tert-butyl-4-hydroxybenzyl)isocyanurate, 1,3,5-tris(3,5-di-tert-butyl-4-hydroxybenzyl)-2,4,6-trimethylbenzene, 1,3,5-tris[(3,5-di-tert-butyl-4-hydroxyphenyl)propionyloxyethyl]isocyanurate, tetrakis[methylene-3-(3',5'-tert-butyl-4'-hydroxyphenyl)propionate]methane, 2-tert-butyl-4-methyl-6-(2-acryloyloxy-3-tert-butyl-5-methylbenzyl)phenol, 3,9-bis[2-(3-tert-butyl-4-hydroxy-5-methylhydrocinnamoyloxy)-1,1-dimethylethyl]-2,4,8,10-tetraoxaspiro[5.5]undecane, triethylene glycol bis[β-(3-tert-butyl-4-hydroxy-5-methylphenyl)propionate], and 3-(3,5-dialkyl-4-hydroxyphenyl)propionic acid derivatives such as stearyl-3-(3,5-di-tert-butyl-4-hydroxyphenyl)propionic acid amide, palmityl-3-(3,5-di-tert-butyl-4-hydroxyphenyl)propionic acid amide, myristyl-3-(3,5-di-tert-butyl-4-hydroxyphenyl)propionic acid amide, and lauryl-3-(3,5-di-tert-butyl-4-hydroxyphenyl)propionic acid amide.

Examples of the phosphorus-based antioxidant include triphenyl phosphite, diisooctyl phosphite, heptakis(dipropylene glycol)triphosphite, triisodecyl phosphite, diphenyl isooctyl phosphite, diisooctyl phenyl phosphite, diphenyl tridecyl phosphite, triisooctyl phosphite, trilauryl phosphite, diphenyl phosphite, tris(dipropylene glycol)phosphite, dioleyl hydrogen phosphite, trilauryl trithiophosphite, bis(tridecyl)phosphite, tris(isodecyl)phosphite, tris(tridecyl)phosphite, diphenyl decyl phosphite, dinonylphenyl bis(nonylphenyl)phosphite, poly(dipropylene glycol)phenyl phosphite, tetraphenyl dipropylene glycol diphosphite, trisnonylphenyl phosphite, tris(2,4-di-tert-butylphenyl)phosphite, tris(2,4-di-tert-butyl-5-methylphenyl)phosphite, tris[2-tert-butyl-4-(3-tert-butyl-4-hydroxy-5-methylphenylthio)-5-methylphenyl]phosphite, tri(decyl) phosphite, octyl diphenyl phosphite, di(decyl)monophenyl phosphite, a mixture of distearyl pentaerythritol and calcium stearate, alkyl (C10) bisphenol A phosphite, tetraphenyl-tetra(tridecyl)pentaerythritol tetraphosphite, bis(2,4-di-tert-butyl-6-methylphenyl)ethyl phosphite, tetra(tridecyl)isopropylidene diphenol diphosphite, tetra(tridecyl)-4,4'-n-butylidenebis(2-tert-butyl-5-methylphenol)diphosphite, hexa(tridecyl)-1,1,3-tris(2-methyl-4-hydroxy-5-tert-butylphenyl)butane triphosphite, tetrakis(2,4-di-tert-butylphenyl)biphenylene diphosphite, 9,10-dihydro-9-oxa-10-phosphaphenanthrene-10-oxide, (1-methyl-1-propenyl-3-ylidene)tris(1,1-dimethylethyl)-5-methyl-4,1-phenylene)hexatridecyl phosphite, 2,2'-methylenebis(4,6-di-tert-butylphenyl)-2-ethylhexyl phosphite, 2,2'-methylenebis(4,6-di-tert-butylphenyl)-octadecyl phosphite, 2,2'-ethylidenebis(4,6-di-tert-butylphenyl)fluorophosphite, 4,4'-butylidenebis(3-methyl-6-tert-butylphenylditridecyl)phosphite, tris(2-[(2,4,8,10-tetrakis-tert-butyldibenzo[d,f][1,3,2]dioxaphosphepin-6-yl)oxy]ethyl)amine, 3,9-bis(4-nonylphenoxy)-2,4,8,10-tetraoxa-3,9-diphosphespiro[5,5]undecane, 2,4,6-tri-tert-butylphenyl-2-butyl-2-ethyl-1,3-propanediol phosphite, poly-4,4'-isopropylidenediphenol C12-15 alcohol phosphite, bis(diisodecyl)pentaerythritol diphosphite, bis(tridecyl)pentaerythritol diphosphite, bis(octadecyl)pentaerythritol diphosphite, bis(nonylphenyl)pentaerythritol diphosphite, bis(2,4-di-tert-butylphenyl)pentaerythritol diphosphite, bis(2,4,6-tri-tert-butylphenyl)pentaerythritol diphosphite, bis(2,6-di-tert-butyl-4-methylphenyl)pentaerythritol diphosphite, and bis(2,4-dicumylphenyl)pentaerythritol diphosphite.

The light stabilizer composition of the present embodiment may be any of a liquid and a solid, and is preferably a liquid. In a case where the light stabilizer composition is a liquid, the light stabilizer composition is excellent in dispersibility and is suitable particularly as a light stabilizer composition for coating materials. In a case where the light stabilizer composition of the present embodiment is a liquid, the light stabilizer composition of the present embodiment can be added with a liquid addition apparatus and thus the light stabilizer composition of the present embodiment is excellent in handleability when used as a light stabilizer composition for coating materials. The light stabilizer composition may also be a masterbatch further including a resin component and a carrier such as silica.

Next, a resin composition of the present embodiment is described.

### <Resin composition>

The resin composition of the present embodiment includes a synthetic resin and the above compound or the above polymer. The resin composition of the present embodiment may include both the above compound and the above polymer.

The resin composition of the present embodiment has excellent weather resistance.

The synthetic resin included in the resin composition is not particularly limited, and may be a thermoplastic resin or a thermosetting resin. Examples of the thermoplastic resin include crystalline resins such as a polyolefin-based resin, a polyamide-based resin, a polyester-based resin, a polyacetal resin, a polylactic acid, and polyphenylene sulfide, amorphous resins such as a polycarbonate resin, a styrene-based resin, an acrylic resin, a urethane-based resin, a halogen-containing resin, a petroleum resin, a coumarone resin, polyvinyl alcohol, polyvinyl acetate, and polyphenylene oxide, and thermoplastic elastomers. Examples of the thermosetting resin include a phenol resin, a urea resin, a melamine resin, an epoxy resin, an unsaturated polyester resin, an alkyd resin, and synthetic rubber. The synthetic resin may be included singly or in combination of two or more kinds thereof. The synthetic resin may be a copolymer or a polymer alloy.

In a case where the resin composition of the present embodiment is a resin composition for coating materials, the synthetic resin preferably includes a heating crosslinking type resin obtained by blending an acrylic resin, a polyester-based resin, an alkyd resin, a urethane-based resin, or the like and a crosslinking agent such as a melamine resin, an epoxy resin, or a polyisocyanate compound, more preferably includes at least one selected from the group consisting of an acrylic melamine resin and a polyester melamine resin, further preferably includes an acrylic melamine resin.

In a case where the resin composition of the present embodiment is a resin composition for molding, the synthetic resin is preferably a thermoplastic resin. The thermoplastic resin is preferably a crystalline resin, more preferably includes at least one selected from the group consisting of a polyolefin-based resin, a polyamide-based resin, a polyester-based resin, a polyacetal resin, a polylactic acid, and polyphenylene sulfide, further preferably includes a polyolefin-based resin. Examples of the polyolefin-based resin include polyethylene-based resins such as low-density polyethylene, linear low-density polyethylene, high-density polyethylene, crosslinked polyethylene and ultrahigh molecular weight polyethylene, polypropylene-based resins such as homopolypropylene, random copolymer polypropylene, block copolymer polypropylene, impact copolymer polypropylene, high-impact copolymer polypropylene and maleic anhydride-modified polypropylene, α-olefin polymers such as polybutene-1, a cycloolefin polymer, poly-3-methyl-1-butene, poly-3-methyl-1-pentene and poly-4-methyl-1-pentene, and α-olefin copolymers such as an ethylene-methyl methacrylate copolymer and an ethylene-vinyl acetate copolymer. The polyolefin-based resin is particularly preferably a polypropylene-based resin from the viewpoint of allowing the resin composition to be more excellent in heat resistance. The molecular weight, the degree of polymerization, the density, the softening point, the proportion of an insoluble fraction in a solvent, the degree of stereoregularity, the presence of a catalyst residue, the type and the blending ratio of each monomer serving as a raw material, the type of a catalyst (for example, a Ziegler catalyst or a metallocene catalyst) used in formation by polymerization, and the like of the polyolefin-based resin are not particularly limited, and are appropriately selected. In a case where the resin composition of the present embodiment is a resin composition for molding, the synthetic resin may include synthetic rubber and/or an elastomer such as a thermoplastic elastomer. In this case, a molded article including the resin composition has excellent impact resistance.

The content of the above compound or the above polymer in the resin composition of the present embodiment may be, for example, 0.001 to 10 parts by mass based on 100 parts by mass of the synthetic resin, and is preferably 0.01 to 5 parts by mass, more preferably 0.1 to 5 parts by mass, further preferably 0.1 to 3 parts by mass.

The resin composition of the present embodiment may include the above-mentioned other hindered amine compound, ultraviolet absorber, benzoate compound, phenol-based antioxidant, phosphorus-based antioxidant, and the like. The resin composition of the present embodiment may further include a sulfur-based antioxidant, other antioxidant, a nucleating agent, a fatty acid metal salt, a flame retardant promotor, a lubricant, a filler, hydrotalcite, an antistatic agent, a fluorescent whitener, a pigment, a dye, and/or the like.

The resin composition of the present embodiment preferably further includes an ultraviolet absorber from the viewpoint of being obtained as a resin composition having further excellent weather resistance. Examples of the ultraviolet absorber include the same as those exemplified for the ultraviolet absorber which may be included in the above light stabilizer composition. Among these ultraviolet absorbers, triazines are particularly preferred. Among triazines, 2-(2-hydroxy-4-hexyloxyphenyl)-4,6-diphenyl-1,3,5-triazine, 2-(2-hydroxy-4-octyloxyphenyl)-4,6-bis(2,4-dimethylphenyl)-1,3,5-triazine, 2-[2-hydroxy-4-{2-(2-ethylhexanoyloxy)ethoxy}phenyl]-4,6-diphenyl-1,3,5-triazine, and 2,4,6-tris(2-hydroxy-4-hexyloxy-3-methylphenyl)-1,3,5-triazine are preferred, 2-(2-hydroxy-4-hexyloxyphenyl)-4,6-diphenyl-1,3,5-triazine and 2-(2-hydroxy-4-octyloxyphenyl)-4,6-bis(2,4-dimethylphenyl)-1,3,5-triazine are more preferred, and 2-(2-hydroxy-4-octyloxyphenyl)-4,6-bis(2,4-dimethylphenyl)-1,3,5-triazine is further preferred. The ultraviolet absorber included in the resin composition may be, for example, in an amount of 0.001 to 10 parts by mass based on 100 parts by mass of the synthetic resin, and is preferably in an amount of 0.01 to 5 parts by mass, more preferably 0.1 to 5 parts by mass, further preferably 0.1 to 3 parts by mass.

### <Method for producing weather-resistant resin composition>

Next, a method for producing a weather-resistant resin composition of the present embodiment is described. The method for producing a weather-resistant resin composition of the present embodiment includes a step of blending the above compound or the above polymer with a synthetic resin. Here, both the above compound and the above polymer may also be blended in the method for producing a weather-resistant resin composition of the present embodiment.

According to the method for producing a weather-resistant resin composition of the present embodiment, a resin composition having excellent weather resistance can be produced.

The above compound is preferably a liquid in the method for producing a weather-resistant resin composition of the present embodiment. In this case, the compound is excellent in dispersibility, thereby enabling a weather-resistant resin composition for coating material compositions, having further excellent light resistance, to be produced. In a case where the above compound is a liquid, the above compound can be blended by use of a liquid addition apparatus, and thus a weather-resistant resin composition for coating material compositions can be efficiently produced.

The method for producing a weather-resistant resin composition of the present embodiment may be, for example, a method including a preparation step of preparing the above compound or the above polymer and, if necessary, other additive, and a blending step of blending each component prepared in the preparation step, with a synthetic resin. The method for blending each component in the blending step is not particularly limited, and examples thereof include a method including adding each component prepared in the preparation step, to a synthetic resin, and then mixing them by use of a mixing apparatus such as an FM mixer, a mill roll, a Banbury mixer, a super mixer, or the like. The method for producing a weather-resistant resin composition of the present embodiment may also be a method including first adding at least one of the above compound or the above polymer and, if necessary, other additive, before polymerization or during polymerization of a synthetic resin monomer, an oligomer or a prepolymer, to produce a synthetic resin polymer, and then adding the remaining component to the synthetic resin polymer obtained. Furthermore, the method for producing a weather-resistant resin composition of the present embodiment may also be a method further including, in addition to the preparation step and the blending step, a melt-kneading step of melt-kneading a mixture obtained in the blending step, by use of a melt-kneading apparatus such as a single-screw extruder or a twin-screw extruder. The melt-kneading temperature in the melt-kneading step may be, for example, 180 to 280°C. The method for producing a weather-resistant resin composition of the present embodiment may also be a method further including a granulating step of granulating the kneaded product obtained in the melt-kneading step. The granulation method is here not particularly limited, and examples thereof include a method using a granulation apparatus such as a pelletizer. The shape of the resin composition obtained by granulation is not particularly limited, and may be, for example, a pellet shape.

### <Method for improving weather resistance of synthetic resin>

Next, a method for improving weather resistance of a synthetic resin of the present embodiment is described. The method for improving weather resistance of a synthetic resin of the present embodiment includes a step of blending the above compound or the above polymer with a synthetic resin. Here, both the above compound and the above polymer may also be blended in the method for improving weather resistance of a synthetic resin of the present embodiment.

According to the method for improving weather resistance of a synthetic resin of the present embodiment, weather resistance of a synthetic resin can be enhanced.

The above compound is preferably a liquid in the method for improving weather resistance of a synthetic resin of the present embodiment. In this case, the compound is excellent in dispersibility, thereby enabling weather resistance of a synthetic resin to be remarkably enhanced particularly in a resin composition for coating material compositions. In a case where the above compound is a liquid, the above compound can be blended by use of a liquid addition apparatus, and thus the method for enhancing weather resistance of the present embodiment is suitable for a resin composition for coating material compositions.

Next, a coating material composition of the present embodiment is described.

### <Coating material composition>

The coating material composition of the present embodiment includes the above resin composition.

The coating material composition of the present embodiment has excellent weather resistance.

The coating material composition may further include, if necessary, a solvent, a curing promotion catalyst, an anti-yellowing agent, a leveling agent, a defoaming agent, a thickening agent, an anti-settling agent, an anti-fogging agent, and/or the like.

The coating material composition of the present embodiment can be suitably used for, for example, painting of automobile exterior components such as bumpers, radiator grilles, front grilles, front panels, fenders, pillars, pillar covers, door mirror stay covers, glass run channels, door mirror housings, lamp housings, wheel covers, spoilers, air spoilers, weatherstrips, window moldings, belt moldings, sunroofs, frontend modules, door modules, backdoor modules, and outside plates, building components such as roofs, walls, floors, and window frames, housings of home appliances such as refrigerators, washers, and vacuum cleaners, and the like.

Next, an article of the present embodiment is described.

### <Article>

The article of the present embodiment includes a coating film obtained by curing the above coating material composition.

The article of the present embodiment has excellent weather resistance.

The article of the present embodiment may be, for example, one which includes a substrate and in which the coating film is stacked on the substrate. The article of the present embodiment may also be one which includes a substrate and an undercoating layer of one, or two or more layers stacked on the substrate and in which the coating film is stacked on the undercoating layer. Furthermore, the article of the present embodiment may also be one which includes a coating layer of one, or two or more layers stacked on the coating film.

Examples of the material of substrate of the article include metals such as zinc-plated copper, zinc alloy-plated steel, stainless steel, and tin-plated steel, and synthetic resins such as an acrylic resin, a polycarbonate resin, a polyester resin, a polystyrene resin, an ABS resin, an AS resin, a polyamide resin, a polyarylate resin, an acrylimide resin, and a polyallyl diglycol carbonate resin.

The coating film can be formed by, for example, coating a substrate or an undercoating layer with the above coating material composition to form a coating material layer, and curing the coating material layer. Examples of the method for coating with the coating material composition include known methods such as brush coating, bar coating, spray coating, dip coating, spin coating, and curtain coating. Examples of the method for curing the coating material layer include known methods such as curing at ordinary temperature, heating curing, and ultraviolet irradiation.

The thickness of the coating film is not particularly limited, and may be, for example, 10 to 300 µm in terms of dry thickness.

Examples of the article of the present embodiment include automobile exterior components such as bumpers, radiator grilles, front grilles, front panels, fenders, pillars, pillar covers, door mirror stay covers, glass run channels, door mirror housings, lamp housings, wheel covers, spoilers, air spoilers, weatherstrips, window moldings, belt moldings, sunroofs, frontend modules, door modules, backdoor modules, and outside plates, building components such as roofs, walls, floors, and window frames, and housings of home appliances such as refrigerators, washers, and vacuum cleaners.

Next, a sealing material of the present embodiment is described.

### <Sealing material>

The sealing material of the present embodiment includes the above resin composition.

The sealing material of the present embodiment has excellent weather resistance.

The sealing material may further include, if necessary, a solvent, a leveling material, a thixotropic agent, a foam stabilizer, a defoaming agent, a foaming agent, a release agent, a latent curing agent, a silane coupling agent, and/or the like. The sealing material of the present embodiment can be preferably used in, for example, applications of automobiles, civil engineering, household equipment, and home appliances.

Next, a molded article of the present embodiment is described.

### <Molded article>

The molded article of the present embodiment is obtained by molding the above resin composition.

The molded article of the present embodiment has excellent weather resistance.

Examples of the molded article of the present embodiment include automobile members such as door moldings, frames for door mirrors, hubcaps, spoilers, bumpers, winker lenses, pillar garnishes, rear finishers, instrument panels, console boxes, meter covers, doorlock bezels, steering wheels, power window switch bases, center clusters, dashboards, side steps, battery cases, luggage cases, front fenders, fender liners, door panels, roof panels, hood panels, trunk lids, backdoor panels, armrests, and air conditioner cases, building materials such as wall materials, floor materials, window frames, wallpaper, and windows, agricultural materials such as houses, tunnels, and flat yarn mesh bags, housings of home appliances such as refrigerators, washers, and vacuum cleaners, industrial materials such as palettes, pail cans, back grind tapes, tapes for liquid crystal protection, and pipes, household products such as tableware, bottle caps, buckets, and bathing products, food packaging materials such as wraps, trays, cups, films, bottles, caps, and storage containers, miscellaneous goods such as toys, storage containers, and synthetic paper, medical products such as medical packs, syringes, catheters, medical tubes, syringe preparations, infusion bags, reagent containers, medicine containers, and individual packages of medicines, 3D printer materials, and fibers such as cloths, woven fabrics, and unwoven fabrics.

Examples of the method for producing the molded article of the present embodiment include an injection molding method, an extrusion method, a blow molding method, a rotational molding method, a vacuum molding method, an inflation molding method, a calendar molding method, a slush molding method, a dip molding method, and a foam molding method.

### EXAMPLES

Hereinafter, the present invention is more specifically described with reference to Examples, but the present invention is not restricted by the following Examples at all.

### <Production of compound>

### (Production Example 1)

A 100-mL three-necked flask was loaded with 5 g of 2,2,6,6-tetramethylpiperidin-4-ol and 15 g of mixed xylene, and 7.1 g of lauroyl chloride was dropped by use of a dropping funnel with heating and stirring at 50°C under a nitrogen atmosphere. After completion of the dropping, the reaction mixture was heated and stirred at 80°C for 2 hours, and subsequently cooled to 50°C. To the reaction mixture were added 0.35 g of sodium carbonate and 12 g of distilled water, and furthermore stirred to neutralize the reaction mixture, thereafter the reaction mixture was left to still stand and separated into an aqueous phase and an organic phase, and the aqueous phase was removed. Subsequently, 12 g of distilled water was added to the organic phase and stirred, thereafter the resultant was left to still stand and separated into an aqueous phase and an organic phase, and the aqueous phase was removed. Subsequently, 4.4 g of n-butyl glycidyl ether and 0.26 g of zinc chloride were added, heated and stirred at 140°C for 6 hours, and thereafter cooled to 50°C. After 12 g of distilled water was added to the reaction mixture and stirred, the resultant was left to still stand and separated into an aqueous phase and an organic phase, and the aqueous phase was removed. Subsequently, the organic phase was concentrated under reduced pressure with a rotary evaporator, and the residue was purified by silica gel column chromatography (developing solvent: hexane/acetone), to obtain 10.8 g of a light yellow liquid.

The light yellow liquid obtained was analyzed by ¹H-NMR (CDCl₃, 400 MHz). The analysis results are shown below.
(¹H-NMR)
δ (v.s.TMS): 5.17 (m, 1H), 3.65 (m, 1H), 3.48 (d, 2H), 3.41 (t, 2H), 2.64 (d, 2H), 2.27 (t, 2H), 1.89 (m, 2H), 1.48-1.66 (m, 6H), 1.43-1.34 (m, 18H), 1.21-1.34 (m, 12H), 0.85-0.95 (t, 6H)

It was confirmed from the analysis results that the light yellow liquid obtained was 1-[3-(n-butyloxy)-2-hydroxypropyl]-2,2,6,6-tetramethylpiperidin-4-yl dodecanoate having the following structure.

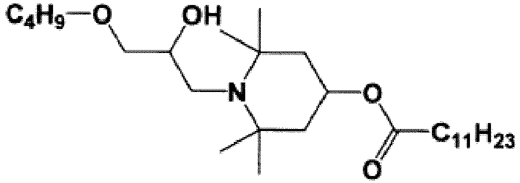

This light yellow liquid was defined as a compound 1.

### (Production Example 2)

The same manner as in Production Example 1 was made except for use of 4.0 g of sebacoyl chloride instead of lauroyl chloride, to obtain 9.2 g of a light yellow liquid.

The light yellow liquid obtained was analyzed by ¹H-NMR (CDCl₃, 400 MHz). The analysis results are shown below.
(¹H-NMR)
δ (v.s.TMS): 5.11 (m, 2H), 3.63 (m, 2H), 3.48 (d, 4H), 3.41 (t, 4H), 2.62 (d, 4H), 2.27 (t, 4H), 1.88 (m, 4H), 1.45-1.66 (m, 12H), 1.22-1.44 (m, 12H), 1.02-1.22 (m, 24H), 0.88-0.95 (t, 6H)

It was confirmed from the analysis results that the light yellow liquid obtained was bis[1-[3-(n-butyloxy)-2-hydroxypropyl]-2,2,6,6-tetramethylpiperidin-4-yl] decanedioate having the following structure.

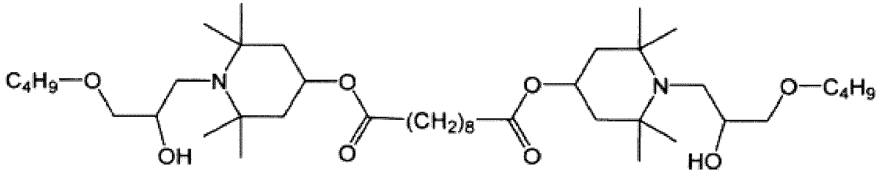

This light yellow liquid was defined as a compound 2.

### (Production Example 3)

A 100-mL three-necked flask was loaded with 5 g of 2,2,6,6-tetramethylpiperidin-4-ol, 4.2 g of n-butyl glycidyl ether, 0.13 g of zinc chloride and 15 g of mixed xylene, heated and stirred at 140°C for 6 hours under a nitrogen atmosphere, and subsequently cooled to 50°C. After 10 g of distilled water was added to the reaction mixture and stirred, the resultant was left to still stand and separated into an aqueous phase and an organic phase, and the aqueous phase was removed. Subsequently, 20 g of mixed xylene was added to dilute the organic phase, and 10.9 g of 3,5,5-trimethylhexanoyl chloride was dropped by use of a dropping funnel with heating and stirring at 50°C under a nitrogen atmosphere. After completion of the dropping, the reaction mixture was heated and stirred at 80°C for 2 hours, and subsequently cooled to 50°C. To the reaction mixture were added 0.7 g of sodium carbonate and 12 g of distilled water, and furthermore stirred to neutralize the reaction mixture, thereafter the reaction mixture was left to still stand and separated into an aqueous phase and an organic phase, and the aqueous phase was removed. Subsequently, 12 g of distilled water was added to the organic phase and stirred, thereafter the resultant was left to still stand and separated into an aqueous phase and an organic phase, and the aqueous phase was removed. Subsequently, the organic phase was concentrated under reduced pressure with a rotary evaporator, and the residue was purified by silica gel column chromatography (developing solvent: hexane/acetone), to obtain 13.3 g of a light yellow liquid.

The light yellow liquid obtained was analyzed by ¹H-NMR (CDCl₃, 400 MHz). The analysis results are shown below.
(¹H-NMR)
δ (v.s.TMS): 5.01-5.12 (m, 2H), 3.34-3.54 (m, 4H)2.73 (d, 2H), 1.96-2.21 (m, 4H), 1.74-1.84 (m, 2H), 1.48-1.58 (m, 2H), 1.20-1.47 (m, 6H), 1.11-1.20 (m, 8H), 1.04-1.11 (m, 6H), 0.96-1.04 (m, 6H), 0.87-0.94 (m, 23H)

It was confirmed from the analysis results that the light yellow liquid obtained was 1-[3-(n-butyloxy)-2-(3,5,5-trimethylhexanoyloxy)propyl]-2,2,6,6-tetramethylpiperidin-4-yl 3,5,5-trimethylhexanate having the following structure.

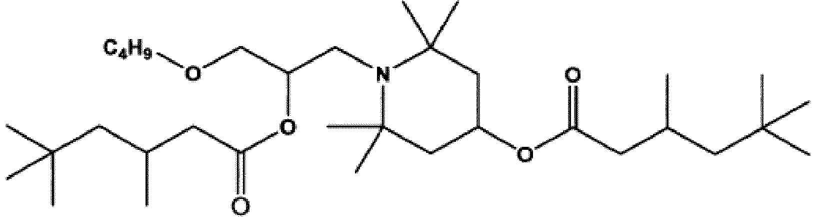

This light yellow liquid was defined as a compound 3.

### (Production Example 4)

The same manner as in Production Example 1 was made except for use of 3.7 g of methacryloyl chloride instead of lauroyl chloride, to obtain 7.9 g of a light yellow liquid.

The light yellow liquid obtained was analyzed by ¹H-NMR (CDCl₃, 400 MHz). The analysis results are shown below.
(¹H-NMR)
δ (v.s.TMS): 6.09 (s, 1H), 5.55 (s, 1H), 5.18 (m, 1H), 3.64 (m, 1H), 3.48( d, 2H), 3.42 (t, 2H), 2.64 (d, 2H), 1.88-2.00 (m, 5H), 1.52-1.63 (m, 4H), 1.32-1.43 (m, 2H), 1.08-1.23 (m, 12H), 0.90-0.95 (t, 3H)

It was confirmed from the analysis results that the light yellow liquid obtained was 1-[3-(n-butyloxy)-2-hydroxypropyl]-2,2,6,6-tetramethylpiperidin-4-yl methacrylate having the following structure.

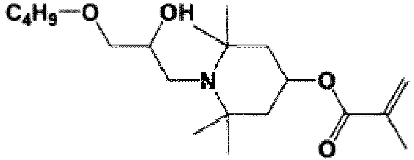

This light yellow liquid was defined as a compound 4.

### (Production Example 5)

The same manner as in Production Example 1 was made except for use of 8.0 g of 3-glysicyloxypropyltrimethoxysilane instead of n-butyl glycidyl ether, to obtain 13.2 g of a light yellow liquid.

The light yellow liquid obtained was analyzed by ¹H-NMR (CDCl₃, 400 MHz). The analysis results are shown below.
(¹H-NMR)
δ (v.s.TMS): 5.17 (m, 1H), 3.95 (tt, 1H), 3.32-3.75 (m, 13H), 2.64 (d, 2H), 2.27 (t, 2H), 1.41-1.95 (m, 8H), 1.03-1.40 (m, 27H), 0.85-0.95 (t, 6H)

It was confirmed from the analysis results that the light yellow liquid obtained was 1-[2-hydroxy-3-(3-(trimethoxysilyl)propoxy)propyl]-2,2,6,6-tetramethylpiperidin-4-yl dodecanoate having the following structure.

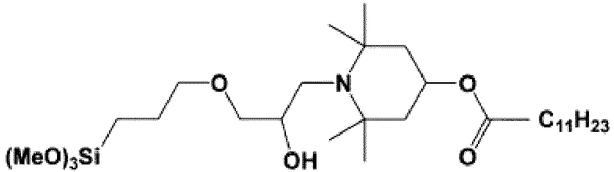

This light yellow liquid was defined as a compound 5.

### (Production Example 6)

The same manner as in Production Example 1 was made except for use of 4.9 g of (R)-glycidyl butyrate instead of n-butyl glycidyl ether, to obtain 11.2 g of a light yellow liquid.

The light yellow liquid obtained was analyzed by ¹H-NMR (CDCl₃, 400 MHz). The analysis results are shown below.
(¹H-NMR)
δ (v.s.TMS): 5.17 (m, 1H), 4.20 (d, 1H), 3.95 (d, 1H), 3.76 (m, 1H), 2.76 (d, 1H), 2.56 (d, 1H), 2.22-2.39 (tt, 4H), 1.40-1.95 (m, 8H), 1.02-1.39 (m, 28H), 0.85-1.02 (t, 6H)

It was confirmed from the analysis results that the light yellow liquid obtained was (R)1-[3-(butanoyloxy)-2-hydroxypropyl]-2,2,6,6-tetramethylpiperidin-4-yl dodecanoate having the following structure.

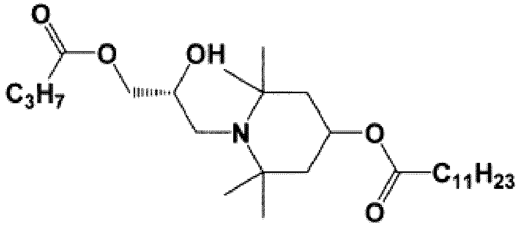

This light yellow liquid was defined as a compound 6.

### (Production Example 7)

The same manner as in Production Example 1 was made except for use of 4.6 g of 1,2-epoxyoctane instead of n-butyl glycidyl ether, to obtain 11.7 g of a light yellow liquid.

The light yellow liquid obtained was analyzed by ¹H-NMR (CDCl₃, 400 MHz). The analysis results are shown below.
(¹H-NMR)
δ (v.s.TMS): 5.17 (m, 1H), 3.43 (m, 1H), 2.68 (d, 2H), 2.29 (t, 2H), 1.87 (m, 2H), 1.21-1.75 (m, 30H), 1.06-1.20 (m, 12H), 0.85-0.95 (t, 6H)

It was confirmed from the analysis results that the light yellow liquid obtained was 1-(2-hydroxyoctyl)-2,2,6,6-tetramethylpiperidin-4-yl dodecanoate having the following structure.

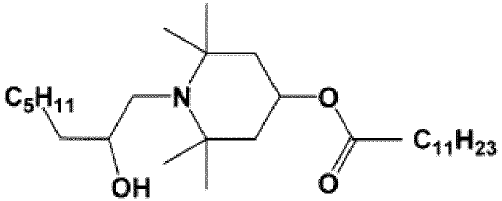

This light yellow liquid was defined as a compound 7.

### <Production of polymer>

### (Production Example 8)

A three-necked flask equipped with a dropping funnel was loaded with 4.0 parts by mass of o-xylene and 0.74 parts by mass of propylene glycol monomethyl ether acetate, and a mixture of 2.6 parts by mass of 2-hydroxyethyl methacrylate, 3.7 parts by mass of isobutyl methacrylate, 7.4 parts by mass of the compound 4 and 0.8 parts by mass of 2,2'-azobisisobutyronitrile was dropped with stirring at 125°C under a nitrogen atmosphere over 2 hours. After completion of the dropping, the reaction solution was stirred at 125°C under a nitrogen atmosphere for 1 hour. After completion of the mixing, 1.5 parts by mass of o-xylene and 3.1 parts by mass of isobutyl acetate were added to the reaction mixture and diluted, to provide a polymer having a resin solid content of 60% by mass, as a slurry. The polymer was defined as a polymer 1.

### <Preparation of coating material composition>

### (Examples 1 to 10)

A three-necked flask equipped with a dropping funnel was loaded with 18.2 parts by mass of o-xylene and 3.4 parts by mass of propylene glycol monomethyl ether acetate, and a mixture of 13.6 parts by mass of a styrene monomer, 0.48 parts by mass of acrylic acid, 22 parts by mass of 2-hydroxypropyl acrylate, 31.6 parts by mass of isobutyl methacrylate and 3.1 parts by mass of 2,2'-azobisisobutyronitrile as a polymerization initiator was dropped with stirring at 125°C under a nitrogen atmosphere over 4 hours. After completion of the dropping, the reaction solution was stirred at 125°C under a nitrogen atmosphere for 1 hour. After completion of the mixing, 2 parts by mass of o-xylene and 14.2 parts by mass of isobutyl acetate were added to the reaction mixture and diluted, to obtain a coating material composition precursor having a resin solid content of 65% by mass, a hydroxyl value (JIS K 0070) of 139, and a weight average molecular weight of 7000. After 50 parts by mass of a n-butanol-modified melamine resin (trade name U-VAN 20SE60 manufactured by Mitsui Chemicals, Inc., resin solid content 60% by mass), the compound 1 to 3, or 5 to 7, or the polymer 1, and an ultraviolet absorber 1 in respective amounts of addition described in Tables 1 and 2, 0.5 parts by mass of p-toluenesulfonic acid as a catalyst, and 0.2 parts by mass of Polyflow KL-400X (manufactured by Kyoeisha Chemical Co., Ltd.) as a leveling agent were added to 70 parts by mass (solid content) of the coating material composition precursor thus obtained, the resultant was diluted with o-xylene and uniformly mixed, and cooled to 25°C, to obtain a coating material composition including a resin composition, having a resin solid content of 50% by mass, and satisfying acrylic resin: melamine resin = 7:3 (mass ratio), as a clear coating material composition. In Tables 1 and 2, the amounts of addition of the compound 1 to 3, 5 to 7 and the ultraviolet absorber 1 are each the amount of addition (parts by mass) based on 100 parts by mass of the resin solid content, and the amount of addition of the polymer 1 is the amount of addition (resin solid content, parts by mass) based on 100 parts by mass of the resin solid content.

Here, the ultraviolet absorber 1 used in the present Examples included 2-(2-hydroxy-4-octyloxyphenyl)-4,6-bis(2,4-dimethylphenyl)-1,3,5-triazine.

### (Comparative Example 1)

The same manner as in Example 1 was made except for no addition of the compound 1, to obtain a coating material composition including a resin composition, having a resin solid content of 50% by mass, and satisfying acrylic resin: melamine resin = 7:3 (mass ratio).

**[Table 1]**

| | Example 1 | Example 2 | Example 3 | Example 4 | Example 5 | Example 6 |
|---|---|---|---|---|---|---|
| Compound 1 | 1 | - | - | - | - | - |
| Compound 2 | - | 1 | - | - | - | - |
| Compound 3 | - | - | - | - | - | - |
| Compound 5 | - | - | - | 1 | - | - |
| Compound 6 | - | - | - | - | 1 | - |
| Compound 7 | - | - | - | - | - | 1 |
| Polymer 1 | - | - | 1 | - | - | - |
| Ultraviolet absorber 1 | | | | | | |
| Retention time of 80% of gloss level | 2160 | 2640 | 2640 | 2160 | 2280 | 2280 |

**[Table 2]**

| | Example 7 | Example 8 | Example 9 | Example 10 | Comparative Example 1 |
|---|---|---|---|---|---|
| Compound 1 | 1 | - | - | - | - |
| Compound 2 | - | 1 | - | - | - |
| Compound 3 | - | - | 1 | - | - |
| Compound 5 | - | - | - | - | - |
| Compound 6 | - | - | - | - | - |
| Compound 7 | - | - | - | - | - |
| Polymer 1 | - | - | - | 1 | - |
| Ultraviolet absorber 1 | 0.5 | 0.5 | 0.5 | 0.5 | - |
| Retention time of 80% of gloss level | 2760 | 2880 | 2640 | 2880 | 1680 |

### <Evaluation of properties of coating material composition>

An epoxy resin-based cationic electrodeposition coating material as a coating material for undercoating was electrodeposited on a surface of a steel plate treated with zinc phosphate, to form an undercoating film having a dry thickness of 20 µm. A polyester melamine resin-based coating material as a coating material for middle coating was painted on the undercoating film thus formed, and baked at 140°C for 20 minutes, to form a middle coating film having a dry thickness of 35 µm. An acrylic melamine resin-based coating material (solid color, white) as a base coating material for topcoating was painted on the middle coating film thus formed, and baked at 140°C for 30 minutes, to form a topcoating base coating film having a dry thickness of 15 µm. The topcoating base coating film thus formed was coated with each coating material composition of Examples 1 to 10 and Comparative Example 1, by use of an automatic applicator equipped with a bar coater, and thereafter baked at 140°C for 30 minutes to cure the coating material composition, thereby forming a coating film having a dry thickness of 40 µm, and the coating film was adopted as a specimen.

The specimen thus obtained was subjected to a weather resistance test by use of a xenon weather meter (apparatus name "Ci4000" manufactured by ATLAS) according to the following conditions.

### (Weather resistance test)

The gloss level of the specimen surface before the weather resistance test was measured in advance, and then the weather resistance test was carried out in conditions in Table 3, according to the standard of ISO4892-2.

**[Table 3]**

| Weather resistance test conditions | |
|---|---|
| Test cycle | Light irradiation for 102 minutes/Light irradiation and water spraying for 18 minutes |
| Irradiance [W/m²] | 0.55 (wavelength 340 nm) |
| Temperature in bath [°C] | 40±2 during light irradiation |
| Black panel temperature [°C] | 65±2 during light irradiation |
| Humidity RH [%] | 50±2 during light irradiation |

The time (h) until the gloss level of the specimen surface after the start of the above test reached 80% of the gloss level of the specimen surface before the weather resistance test was set as "retention time of 80% of gloss level", and adopted as an index of weather resistance. The results are described together in Tables 1 and 2.

The gloss level of the specimen surface was measured with a glossimeter (apparatus name VG-2000 manufactured by Nippon Denshoku Industries Co., Ltd.) in a condition of an incident angle of 20°, according to the standard of JIS Z8741.

It was found from the results shown in Tables 1 and 2 that each of the coating material compositions of Examples 1 to 10 had excellent weather resistance as compared with the coating material composition of Comparative Example 1.

### <Production of molded article>

### (Example 11)

After 0.1 parts by mass of tetrakis[methylene-3-(3',5'-tert-butyl-4'-hydroxyphenyl)propionate]methane, 0.1 parts by mass of tris(2,4-di-tert-butylphenyl)phosphite, 0.05 parts by mass of calcium stearate, and 0.05 parts by mass of the compound 1 were blended based on 100 parts by mass of homopolypropylene (having a MFR of 10 g/10 min at a temperature 230°C and a load of 2.16 kg), and uniformly mixed by use of a Henschel mixer, the blended product was melt-kneaded with a twin-screw extruder (TEX28V manufactured by JAPAN STEEL WORKS, LTD.) in conditions of a solvent temperature of 230°C and a screw speed of 150 rpm, and granulated, to obtain a resin composition pellet. The resin composition pellet obtained was dried at 60°C for 8 hours, and thereafter injection molded with an injection molding machine (EC100-2A manufactured by Toshiba Machine Group Engineering) in molding conditions of a resin temperature of 230°C and a mold temperature of 40°C, to produce a rectangular sheet-shaped test piece of a dimension of 6.0 cm × 2.7 cm × 2 mm.

### (Comparative Example 2)

The same manner as in Example 11 was made except for no blending of the compound 1, to obtain a rectangular sheet-shaped test piece of a dimension of 6.0 cm × 2.7 cm × 2 mm.

### <Evaluation of properties of molded article>

Each test piece of Example 11 and Comparative Example 2 was adopted and the gloss level of the test piece surface before the weather resistance test was measured in advance, and then the weather resistance test was carried out in conditions of an intensity of irradiation at a wavelength of 340 nm, of 0.55 W/m², continuous irradiation, a black panel temperature of 89°C±3°C, and no rainfall, by use of a xenon weather meter (apparatus name "Ci4000" manufactured by ATLAS), according to the standard of SAEJ1885(J2412). The time (h) until the gloss level of the test piece surface after the start of the test reached 80% of the gloss level of the test piece surface before the weather resistance test was set as "retention time of 80% of gloss level", and adopted as an index of weather resistance.

As a result, the retention time of 80% of gloss level of the test piece of Example 11 was 780 h. On the other hand, the retention time of 80% of gloss level of the test piece of Comparative Example 2 was 120 h.

It has been confirmed from the foregoing that the compound of the present invention can impart excellent weather resistance to a resin composition.

## Claims

1. A compound represented by the following general formula (1'): 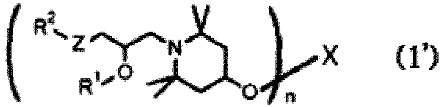 wherein n represents 1 or 2, when n is 1, X represents a substituted or unsubstituted monovalent hydrocarbon group having 1 to 30 carbon atoms, a group represented by -C(=O)-R³, or a group represented by the following general formula (2), when n is 2, X represents a substituted or unsubstituted divalent hydrocarbon group having 1 to 30 carbon atoms, a group represented by -C(=O)-Y-C(=O)-, or a -C(=O)- group and Y represents a single bond, or a substituted or unsubstituted divalent hydrocarbon group having 1 to 30 carbon atoms, Z represents a single bond or an oxygen atom, R¹ represents a hydrogen atom, a substituted or unsubstituted monovalent hydrocarbon group having 1 to 30 carbon atoms, or a group represented by -C(=O)-R⁵, R² represents a substituted or unsubstituted monovalent hydrocarbon group having 1 to 30 carbon atoms, or a group represented by -C(=O)-R⁶, and each of R³, R⁵ and R⁶ independently represents a substituted or unsubstituted monovalent hydrocarbon group having 1 to 30 carbon atoms; 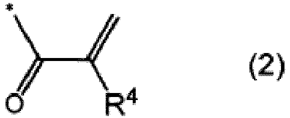 wherein * represents a site bound with an oxygen atom and R⁴ represents a hydrogen atom, or a substituted or unsubstituted monovalent hydrocarbon group having 1 to 30 carbon atoms.

2. The compound according to claim 1, wherein, in the general formula (1'), n represents 1 or 2, when n is 1, X represents a substituted or unsubstituted monovalent aliphatic hydrocarbon group having 1 to 30 carbon atoms, a group represented by -C(=O)-R³, or the group represented by the general formula (2), when n is 2, X represents a substituted or unsubstituted divalent aliphatic hydrocarbon group having 1 to 30 carbon atoms, a group represented by -C(=O)-Y-C(=O)-, or a -C(=O)- group and Y represents a single bond, or a substituted or unsubstituted divalent aliphatic hydrocarbon group having 1 to 30 carbon atoms, Z represents a single bond or an oxygen atom, R¹ represents a hydrogen atom, a substituted or unsubstituted monovalent aliphatic hydrocarbon group having 1 to 30 carbon atoms, or a group represented by -C(=O)-R⁵, R² represents a substituted or unsubstituted monovalent aliphatic hydrocarbon group having 1 to 30 carbon atoms, or a group represented by -C(=O)-R⁶, and each of R³, R⁵ and R⁶ independently represents a substituted or unsubstituted monovalent aliphatic hydrocarbon group having 1 to 30 carbon atoms, and in the general formula (2), * represents a site bound with an oxygen atom and R⁴ represents a hydrogen atom, or a substituted or unsubstituted monovalent aliphatic hydrocarbon group having 1 to 30 carbon atoms.

3. The compound according to claim 1, wherein Z is an oxygen atom.

4. The compound according to claim 2, wherein Z is an oxygen atom.

5. The compound according to claim 1, represented by the following general formula (1): 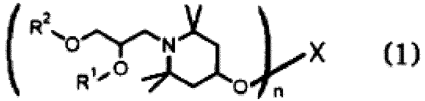 wherein n represents 1 or 2, when n is 1, X represents a monovalent hydrocarbon group having 1 to 30 carbon atoms, a group represented by -C(=O)-R³, or a group represented by the following general formula (2), when n is 2, X represents a divalent hydrocarbon group having 1 to 30 carbon atoms, a group represented by -C(=O)-Y-C(=O)-, or a -C(=O)- group and Y represents a single bond or a divalent hydrocarbon group having 1 to 30 carbon atoms, R¹ represents a hydrogen atom, a monovalent hydrocarbon group having 1 to 30 carbon atoms or a group represented by -C(=O)-R⁵, and each of R², R³ and R⁵ independently represents a monovalent hydrocarbon group having 1 to 30 carbon atoms, 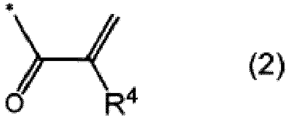 wherein * represents a site bound with an oxygen atom and R⁴ represents a hydrogen atom, or a monovalent hydrocarbon group having 1 to 30 carbon atoms.

6. The compound according to claim 5, wherein, in the general formula (1), n represents 1 or 2, when n is 1, X represents a monovalent aliphatic hydrocarbon group having 1 to 30 carbon atoms, a group represented by -C(=O)-R³, or the group represented by the general formula (2), when n is 2, X represents a divalent aliphatic hydrocarbon group having 1 to 30 carbon atoms, a group represented by -C(=O)-Y-C(=O)-, or a -C(=O)- group and Y represents a single bond or a divalent aliphatic hydrocarbon group having 1 to 30 carbon atoms, R¹ represents a hydrogen atom, a monovalent aliphatic hydrocarbon group having 1 to 30 carbon atoms or a group represented by -C(=O)-R⁵, and each of R², R³ and R⁵ independently represents a monovalent aliphatic hydrocarbon group having 1 to 30 carbon atoms, and in the general formula (2), * represents a site bound with an oxygen atom and R⁴ represents a hydrogen atom or a monovalent aliphatic hydrocarbon group having 1 to 30 carbon atoms.

7. A polymer obtained by polymerizing a monomer component comprising the compound according to any one of claims 1 to 6 in which n is 1 and X is the group represented by the general formula (2).

8. A light stabilizer composition comprising the compound according to any one of claims 1 to 6.

9. The light stabilizer composition according to claim 8, wherein the composition is a liquid.

10. A light stabilizer composition comprising the polymer according to claim 7.

11. A resin composition comprising a synthetic resin, and the compound according to any one of claims 1 to 6 or the polymer according to claim 7.

12. A coating material composition comprising the resin composition according to claim 11.

13. An article comprising a coating film obtained by curing the coating material composition according to claim 12.

14. A sealing material comprising the resin composition according to claim 11.

15. A molded article obtained by molding the resin composition according to claim 11.

16. A method for producing a weather-resistant resin composition, comprising a step of blending the compound according to any one of claims 1 to 6 or the polymer according to claim 7, with a synthetic resin.

17. A method for improving weather resistance of a synthetic resin, comprising a step of blending the compound according to any one of claims 1 to 6 or the polymer according to claim 7, with the synthetic resin.
